# EUROPEAN PATENT SPECIFICATION

(11) **EP 2 283 043 B1**
(45) Date of publication and mention of the grant of the patent: **13.08.2014**
(21) Application number: 09730330.9
(22) Date of filing: 06.04.2009
(51) Int. Cl.: C12N 5/00

(54) **METHODS OF RECOMBINANT PRODUCTION OF GLYCOPROTEINS**
VERFAHREN ZUR REKOMBINANTEN HERSTELLUNG VON GLYCOPROTEINEN
PROCÉDÉS DE PRODUCTION RECOMBINANTE DE GLYCOPROTÉINES

(30) Priority: 07.04.2008 US 42975 P
(43) Date of publication of application: 16.02.2011
(73) Proprietor: Bayer HealthCare, LLC, 100 Bayer Boulevard Whippany, New Jersey 07981-0915 (US)
(72) Inventor: WANG, Frank, S., Zhonghe Dist., New Taipei City 235 Taiwan (TW); WANG, Jin, Lafayette CA 94549 (US)
(74) Representative: BIP Patents
(86) International application number: PCT/US2009/039633
(87) International publication number: WO 2009/126564

(56) References cited:
- EP-A2- 1 096 017
- WO-A1-01/59075
- WO-A1-96/39488
- US-B1- 6 528 286
- US-B2- 6 528 283
- NOK ANDREW J ET AL: "Characterization of sialidase from Entamoaeba hystolitica and possible pathogenic role in amebiasis.", PARASITOLOGY RESEARCH MAR 2003 LNKD- PUBMED:12632168, vol. 89, no. 4, March 2003 (2003-03), pages 302-307, XP002628195, ISSN: 0932-0113
- HIRAIWA ET AL.: 'Activation of Human Lysosomal Sialidase' J. BIOCHEM. vol. 114, 1993, pages 901 - 905, XP008146092
- FANZANI ET AL.: 'Insulin-like growth factor 1 signaling regulates cytosolic sialidase Neu2 expression during myoblast differentiation and hypertrophy' FEBS JOUMAL vol. 273, 2006, pages 3709 - 3721, XP008146076
- PEPYS ET AL.: 'Human serum amyloid P component is an invariant constituent of amyloid deposits and has a uniquely homogeneous glycostructure' PROC. NATI. ACAD. SCI. USA vol. 91, no. 12, June 1994, pages 5602 - 5606, XP008146095
- BOYSEN ET AL.: 'Recombinant human serum amyloid P component from Pichia pastoris: production and characterization' PROTEIN EXPRESSION AND PURIFICATION vol. 35, no. 2, 2004, pages 284 - 292, XP004506564

## Description

### FIELD OF THE INVENTION

The invention relates to methods of recombinant production of glycoproteins in eukaryotic cell culture. Provided are methods of improving the glycosylation pattern in recombinantly produced glycoproteins by increasing the percentage of oligosaccharides terminating in sialic acid.

### BACKGROUND

Glycoproteins generally refer to peptides and proteins having more than about ten amino acids and at least one oligosaccharide side chain. The carbohydrate composition of an attached oligosaccharide can affect a glycoprotein's solubility, biological activity, resistance to protease degradation, and *in vivo* circulation. The terminal residues of glycans are particularly important for therapeutic proteins since the final sugar moiety often controls their *in vivo* arculatory half-life. Glycoproteins with oligosaccharides terminating in sialic acid typically remain in circulation longer due to the presence of receptors in macrophages and hepatocytes that bind and rapidly degrade structures terminating in other sugars/amino-sugars from the bloodstream.

It has been found that copper (Cu²⁺) ions can increase sialic acid capping (see U.S. Pat. No. 6,528,286 to Ryll), however, it is also known that eukaryotic cells have been shown to be sensitive to Cu²⁺ concentration in culture media. Thus, there is a need to provide a safer alternative to Cu²⁺ for increasing sialic acid capping of glycoproteins.

Accordingly, provided herein are methods and processes to increase the percentage of oligosaccharides terminating in sialic acid in order to extend the half-life of the glycoprotein.

### DESCRIPTION OF THE DRAWINGS

***Figure 1*** shows the methodology used for calculation of glycoprotein capping percentage. Figure 1(A) is a diagram of a protein which has been glycosylated, some of which have been capped by sialic acid. Figure 1(B) shows a flowchart of the method used to calculate sialic acid capping. Figure 1 (C) shows the formula used for capping percentage.
***Figure 2*** depicts a graph showing capping trends of recombinant glycoprotein K in commercial-scale manufacturing campaigns.
***Figure** 3* depicts a graph showing sialidase activity of cell lysate from commercial-scale manufacturing campaigns.
***Figure 4*** depicts graphs showing sialidase activity of a low capping cell source and high capping cell source during 15L bioreactor fermentation. Figure 4(A) shows free sialidase activity in the supernatant. Figure 4(B) shows sialidase activity in cell lysates.
***Figure 5*** provides a diagram of the Insulin-like growth factor 1 (IGF-1) and insulin receptor signaling pathway.
***Figure 6*** depicts that sialidase regulation is mediated via IGF-1/Insulin receptor pathway. Figure 6(A) is an immunoblot of phosphorylated Akt. Figure 6(B) is an immunoblot of phosphorylated Erk. Figure 6(C) is a graph showing intracellular sialidase activity with different inhibitors.
***Figure 7*** shows the effect of insulin and IGF-1 on sialidase expression. Figure 7(A) is an immunoblot of sialidase. Figure 7(B) is a graph showing intracellular sialidase activity BHK cells grown in media with insulin, media with reduced insulin (1/10^{th} the concentration), and media with reduced insulin + IGF-1.
***Figure 8*** shows that sialidase activity in BHK cells is pH dependent.
***Figure 9*** depicts a graph showing the inhibition of sialidase activity using trace elements, including CaCl₂, NiCl₂, CoCl₂, and ZnCl₂.

### SUMMARY

Provided are methods and processes for producing glycoproteins by eukaryotic cell culture which provide a glycoprotein product containing oligosaccharides terminating in one or more sialic residues. The cell culture processes described herein allow for the recovery of a glycoprotein product whose oligosaccharides are not compromised by degradative events associated with standard cell culture procedures. The processes described herein overcome the problem of desialylation of a glycoprotein's oligosaccharide side chains that are associated with standard glycoprotein production methods. The invention provides economic and commercial benefits through the recovery of greater useful quantities of glycoprotein product.

Accordingly, provided are processes for producing glycoproteins by eukaryotic and especially mammalian cell culture comprising culturing a host cell expressing a glycoprotein in the presence of zinc or cobalt ions in a cell culture medium in a concentration effective to minimize the loss of sialic acid from the oligosaccharides. The present invention therefore provides various cell culture processes to preserve particular glycoforms of glycoproteins produced in mammalian cell culture. In some embodiments, the zinc or cobalt ions are administered to the cell culture medium or to cell lysate at levels sufficient to inhibit sialidase activity.

Provided are, in some embodiments, methods or processes for producing a glycoprotein in mammalian cell culture by adding an effective amount of Co²⁺ to a culture medium in which cells producing the glycoprotein are grown. In some embodiments, the effective amount is enough to inhibit sialidase expression and/or activity. Thus, in some embodiments, the concentration of Co²⁺ is between approximately 0.005 mM and 50 mM. The foregoing parameter is controlled to affect the mature glycoprotein sialic acid content. In some embodiments, the Co²⁺ is derived from a cobalt salt. In some embodiments, the Co²⁺ is derived from compounds selected from the group consisting of CoCl₂ and CoSO₄.

The present invention provides, in some embodiments, methods or processes for producing a glycoprotein in mammalian cell culture by adding an effective amount of Zn²⁺ to a culture medium in which cells producing the glycoprotein are grown to inhibit sialidase expression and/or activity. In some embodiments, the effective amount is enough to inhibit sialidase expression and/or activity. Thus, in some embodiments, the concentration of Zn²⁺ is between approximately 0.005 mM and 50 mM. The foregoing parameter is controlled to affect the mature glycoprotein sialic acid content. In some embodiments, the Zn²⁺ is derived from a zinc salt. In some embodiments, the Zn²⁺ is derived from compounds selected from the group consisting of ZnCl₂ and ZnSO₄.

Also provided in some embodiments are methods or processes for producing a glycoprotein in a mammalian cell culture, such as in a baby hamster kidney (BHK) cell culture or Chinese hamster ovary (CHO) cell culture, by adding an effective amount of insulin-like growth factor 1 (IGF-1) to the cell culture medium. In some embodiments, the levels of IGF-1 useful to reduce expression and/or activity of sialidase is selected from the following ranges: 1-90 ng/ml, 1-10 ng/ml, 1-5 ng/ml, 5-10 ng/ml, 10-20 ng/ml, 10-15 ng/ml, 15-20 ng/ml, 20-30 ng/ml, 20-25 ng/ml, 25-30 ng/ml, 30-40 ng/ml, 30-35 ng/ml, 35-40 ng/ml, 40-50 ng/ml, 40-45 ng/ml, 45-50 ng/ml, 50-60 ng/ml, 60-70 ng/ml, 70-80 ng/ml, and 80-90 ng/ml.

In some embodiments, the methods can be further optimized by a combination of techniques used above to increase sialic acid capping. In some embodiments, zinc ions and IGF-1 can be added to the cell culture medium. In some embodiments, cobalt ions and IGF-1 can be added to the cell culture medium. In other embodiments, zinc and cobalt ions can be added to the cell culture medium. In some embodiments, zinc ions, cobalt ions or IGF-1 can be added to the cell culture medium with other enhancers known in the art.

Additionally, the methods can be further optimized by altering the pH of the cell culture medium to reduce the sialidase activity. In some embodiments, particularly for BHK cells, the pH is 6.0 or below, 6.5 or below, 7.0 or below, 6.0 to 7.0, and or 6.5 to 7.0.

The methods and processes are believed to be useful with any recombinantly produced glycoprotein.

Also provided is a process for producing glycoprotein by mammalian cell culture, the process comprising: culturing a mammalian host cell in a medium in a production phase which is characterized by adding an effective amount of zinc ion to the cell culture, said effective amount of zinc ion in a concentration effective to minimize the loss of sialic acid on the glycoprotein.

Also provided is a process for producing glycoprotein by mammalian cell culture, the process comprising: culturing a mammalian host cell in a medium in a production phase which is characterized by adding an effective amount of cobalt ion to the cell culture, said effective amount of cobalt ion in a concentration effective to minimize the loss of sialic acid on the glycoprotein.

Also provided is a process for producing glycoprotein by mammalian cell culture, the process comprising: culturing a mammalian host cell in a medium in a production phase which is characterized by adding an effective amount of insulin-like growth factor 1 (IGF-1) to the cell culture, said effective amount of cobalt ion in a concentration effective to minimize the loss of sialic acid on the glycoprotein.

### DETAILED DESCRIPTION

It is to be understood that this invention is not limited to the particular methodology, protocols, cell lines, animal species or genera, constructs, and reagents described and as such may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a," "and," and "the" include plural reference unless the context clearly dictates otherwise. Thus, for example, reference to "an oligosaccharide" is a reference to one or more oligosaccharide and includes equivalents thereof known to those skilled in the art, and so forth.

Unless defined otherwise, all technical and scientific terms used herein have the same meaning as commonly understood to one of ordinary skill in the art to which this invention belongs. Although any methods, devices, and materials similar or equivalent to those described herein can be used in the practice or testing of the invention, the preferred methods, devices and materials are now described.

### I. Definitions

The carbohydrate moieties of the present invention will be described with reference to commonly used nomenclature for the description of oligosaccharides. A review of carbohydrate chemistry which uses this nomenclature is found in Hubbard and Ivatt (1981) Ann. Rev. Biochem. 50:555-583. This nomenclature includes, for instance, Man, which represents mannose; GlcNAc, which represents 2-N-acetylglucosamine; Gal which represents galactose; and Glc, which represents glucose. Sialic acids (SA's) are described with reference to the shorthand notation NeuNAc, for 5-N-acetylneuraminic acid, and NeuNGc for 5-glycolylneuraminic acid (J. Biol. Chem, 1982 257:3347; J. Biol. Chem., 1982, 257:3352).

As used herein "glycoprotein" refers generally to peptides and proteins having more than about ten amino acids and at least one oligosaccharide side chain. The glycoproteins may be homologous to the host cell, or preferably, they are heterologous, i.e., foreign, to the host cell being utilized, such as a human protein produced by a Chinese hamster ovary (CHO) cell or baby hamster kidney (BHK) cell. In some embodiments, mammalian glycoproteins (glycoproteins that were originally derived from a mammalian organism) are used, and in some embodiments, those which are directly secreted into the medium. Examples of mammalian glycoproteins include molecules such as cytokines and their receptors, as well as chimeric proteins comprising cytokinxes or their receptors, including, for instance tumor necrosis factor alpha and beta, their receptors (TNFR-1; EP 417,563 published Mar. 20, 1991; and TNFR-2, EP 417,014 published Mar. 20, 1991) and their derivatives, for example, a tumor necrosis factor receptor-immunoglobulin chimera; renin; a growth hormone, including human growth hormone, and bovine growth hormone; growth hormone releasing factor, parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VII, factor VIII, factor IX, tissue factor, and von Willebrands factor, anti-dotting factors such as Protein C; atrial natriuretic factor, lung surfactant; a plasminogen activator, such as urokinase or human urine or tissue-type plasminogen activator (t-PA); bombesin; thrombin, hemopoietic growth factor; enkephalinase; RANTES (regulated on activation normally T-cell expressed and secreted); human macrophage inflammatory protein (MIP-1-alpha); a serum albumin such as human serum albumin; mullerian-inhibiting substance; relaxin A-chain; relaxin B-chain; prorelaxin; mouse gonadotropin-associated peptide; a microbial protein, such as beta-lactamase; DNase; inhibin; activin; vascular endothelial growth factor (VEGF); receptors for hormones or growth factors; integrin; protein A or D; rheumatoid factors; a neurotrophic factor such as bone-derived neurotrophic factor (BDNF), neurotrophin-3,-4,-5, or-6 (NT-3, NT-4, NT-5, or NT-6), or a nerve growth factor such as NGF-β; platelet-derived growth factor (PDGF); fibroblast growth factor such as aFGF and bFGF; epidermal growth factor (EGF); transforming growth factor (TGF) such as TGF-alpha and TGF-beta, including TGF-β1, TGF-β2. TGF-β3, TGF-β4, or TGF-β5; insulin-like growth factor-I and-II (IGF-I and IGF-II); des(1-3)-IGF-I (brain IGF-1), insulin-like growth factor binding proteins; CD proteins such as CD-3, CD-4, CD-8, and CD-19; erythropoietin; osteoinductive factors; immunotoxins; a bone morphogenetic protein (BMP); an interferon such as interferon-alpha,-beta, and-gammna; colony stimulating factors (CSFs), e.g., M-CSF, GM-CSF, and G-CSF; interleukins (TLs), e.g., IL-1 to IL-10; superoxide dismutase; T-cell receptors; surface membrane proteins; decay accelerating factor, viral antigen such as, for example, a portion of the AIDS envelope; transport proteins; homing receptors; addressings regulatory proteins; antibodies; chimeric proteins, such as immunoadhesins (immunoadhesins are described in, for example, U.S. Pat. Nos. 5,116,964, 5,714,147 and 5,336,603 immunoadhesons include CD4 (Capon et al. , (1989) Nature 337:525-531; Traunecker et al, (1989) Nature 339:68-70; and Byrn et al., (1990) Nature 344:667-670); L-selectin or homing receptor (Watson et al., (1990) J. Cell. Biol. 110:2221-2229; and Watson et al., (1991) Nature 349:164-167); CD44 (Aruffo et al., (1990) Cell 61:11303-1313; CD28 and B7 (Linsley et al., (1991) J. Exp. Med. 173:721-730); CTLA-4 (Linsley et al., J. Exp. Med. 174:561-569); CD22 (Stamenkovic et al., Cell 66:1133-1144); TNF receptor (Ashkenazi et al., (1991) Proc. Natl. Acad. Sci. USA 88:10535-10539; Lesslauer et al., (1991) Eur J. Immunol. 27:2883-2886; and Peppel et al., (1991) J. Exp Med. 174:1483-1489); NP receptors (Bennett et al., (1991) J. Biol. Chem. 266:23060-23067; interferon γ receptor (Kurschner et al., (1992) J. Biol. Chem. 267:9354-9360; 4-1BB (Chalupny et al., (1992) PNAS USA 89:10360-10364) and IgE receptor α (Ridgway and Gorman, (1991) J. Cell. Biol.115, Abstract No. 1448)) and fragments of any of the above-listed polypeptides.

The term "antibody" is used in the broadest sense and specifically covers single monoclonal antibodies (including agonist and antagonist antibodies) and antibody compositions with polyepitopic specificity. The term "antibody" specifically covers monoclonal antibodies (including full length monoclonal antibodies), polyclonal antibodies, multispecific antibodies (e.g., bispecific antibodies), and antibody fragments.

The terms "cell culture medium" and "culture medium" and "fermentation medium" refer to a nutrient solution used for growing mammalian cells that typically provides at least one component from one or more of the following categories:
1) an energy source, usually in the form of a carbohydrate such as glucose;
2) all essential amino acids, and usually the basic set of twenty amino acids plus cysteine;
3) vitamins and/or other organic compounds required at low concentrations;
4) free fatty acids; and
5) trace elements, where trace elements are defined as inorganic compounds or naturally occurring elements that are typically required at very low concentrations, usually in the micromolar range.

The nutrient solution may optionally be supplemented with one or more components from any of the following categories:
1) hormones and other growth factors as, for example, insulin, transferrin, and epidermal growth factor,
2) salts and buffers as, for example, calcium, magnesium, and phosphate;
3) nucleosides and bases such as, for example, adenosine, thymidine, and hypoxanthine; and
4) protein and tissue hydrolysates.

The cell culture medium is generally "serum free", when the medium is essentially free of serum from any mammalian source (e.g. fetal bovine serum [FBS]). By "essentially free" is meant that the cell culture medium comprises between about 0-5% serum, preferably between about 0-1% serum and most preferably between about 0-0.1% serum.

The term "mammalian host cell", "host cell", "mammalian cell" and the like, refer to cell lines derived from mammals that are capable of growth and survival when placed in either monolayer culture or in suspension culture in a medium containing the appropriate nutrients and growth factors. The necessary growth factors for a particular cell line are readily determined empirically without undue experimentation, as described for example in Mammalian Cell Culture, Mather, J. P. ed., Plenum Press, N.Y. (1984), and Bames and Sato, (1980) Cell, 22:649. Typically, the cells are capable of expressing and secreting large quantities of a particular glycoprotein of interest into the culture medium. Examples of suitable mammalian host cells within the context of the present invention may include baby hamster kidney (BHK) cells, Chinese hamster ovary (CHO) cells, human hybrid host cells such as HKB cells (see U.S. Patent No. 6,136,599, cells made via fusion of human embryonic kidney cells (293S) and modified Burkitt's lymphoma cells (2B8)). CHO/-DHFR (CHO, Urlaub and Chasin, Proc. Natl. Acad. Sci. USA, 77:4216 (1980)); dp12.CHO cells (EP 307,247 published Mar. 15, 1989); mouse sertoli cells (TM4, Mather, Biol. Reprod., 23:243-251 (1980)); human cervical carcinoma cells (HELA, ATCC CCL 2); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68 [1982]); MRC 5 cells; FS4 cells.

"Growth phase" of the cell culture refers to the period of exponential cell growth (the log phase) where cells are generally rapidly dividing. During this phase, cells are cultured for a period of time, usually between 1-5 days, and under such conditions that cell growth is maximized. The determination of the growth cycle for the host cell can be determined for the particular host cell envisioned without undue experimentation. "Period of time and under such conditions that cell growth is maximized" and the like, refer to those culture conditions that, for a particular cell line, are determine to be optimal for cell growth and divisions. During the growth phase, cells are cultured in nutrient medium containing the necessary additives generally at about 30°-40° C., preferably at about 37° C., in a humidified, controlled atmosphere, such that optimal cell growth is achieved for a particular cell line. Cells are maintained in the growth phase for a period of about between one and five days, usually between two to three days.

"Transition phase" of the cell culture refers to the period of time during which culture conditions for the production phase are engaged. During the transition phase environmental factors such as zinc or cobalt ion concentration and temperature are shifted from growth conditions to production conditions.

"Production phase" of the cell culture refers to the period of time during which cell growth has plateaued or is maintained at a near constant level. During the production phase, logarithmic cell growth has ended and protein production is primary. During this period of time the medium is generally supplemented to support continued protein production and to achieve the desired glycoprotein product.

The term "expression" or "expresses" are used herein to refer to transcription and translation occurring within a host cell. The level of expression of a product gene in a host cell may be determined on the basis of either the amount of corresponding mRNA that is present in the cell or the amount of the protein encoded by the product gene that is produced by the cell. For example, mRNA transcribed from a product gene is desirably quantitated by northern hybridization. Sambrook et al., Molecular Cloning: A Laboratory Manual, pp. 7.37.57 (Cold Spring Harbor Laboratory Press, 1989). Protein encoded by a product gene can be quantitated either by assaying for the biological activity of the protein or by employing assays that are independent of such activity, such as western blotting or radioimmunoassay using antibodies that are capable of reacting with the protein. Sambrook et al., Molecular Cloning: A Laboratory Manual, pp. 18.1-18.88 (Cold Spring Harbor Laboratory Press, 1989).

As used herein, the terms "protein", "peptide" and "polypeptide" are used interchangeably to denote an amino acid polymer or a set of two or more interacting or bound amino acid polymers.

### II. Cell Culture Procedures

It has been determined that providing certain trace elements, such as zinc and cobalt, or growth factors, such as insulin-like growth factor 1 (IGF-1), in a glycoprotein-producing eukaryotic host cell culture medium results in a glycoprotein product with increased sialic acid content in the oligosaccharide side chain. Since proteins expressing one or more sialic acid residues per complex oligosaccharide structure may have longer clearance rates in vivo the clearance rate of the glycoprotein produced may be manipulated within broad limits by the overall degree of sialylation of the preparation. The present invention provides for processes for increasing sialylation of a glycoprotein that can be recovered from a eukaryotic and especially a mammalian cell culture.

In some embodiments, eukaryotic cells, especially mammalian cells, are cultured to produce a desired glycoprotein product. In choosing a host cell for the production of the glycoprotein within the context of the present invention, it is important to recognize that different host cells have characteristic and specific mechanisms for the translational and post-translational processing and modification (e.g., glycosylation, cleavage) of the proteins expressed. Appropriate cell lines should be chosen to ensure that the desired post translational modifications are possible. Alternatively, host cells can be modified to express a desired gene product required for the specific post-translational modification.

In particular, the mammalian cells that express the desired protein should express or be manipulated to express the particular enzymes such that under the appropriate conditions, described herein, the appropriate post translational modification occurs in vivo. The enzymes include those enzymes necessary for the addition and completion of N-and O-linked carbohydrates such as those described in Hubbard and Ivan Suira for N-linked oligosaccharides. The enzymes optionally include oligosaccharyltransferase, alpha-glucosidase I, alpha-glucosidase II, ER alpha(1,2)mannosidase, Golgi alpha-mannodase I, N-acetylyglucosaminyltransferase I, Golgi alpha-mannodase II, N-acetylyglucosaminyltransferase II, alpha(1,6)fucosyltransferase, and β (1,4)galactosyltranferase. Additionally, the host cell expresses the appropriate sialyl transferase that can be expected to attach the terminal sialic acid in specific position and linkage as part of the host cell genome. Optionally, the host cell can be made to express the appropriate sialyl transferases by, for instance, transfection of the host cell with the DNA encoding the sialyl tranferase.

The sialyl transferases described above would be expected to add the terminal sialic acid residue to the appropriate oligosaccharide core structure such as Galβ1-4GlcNAc. Appropriate sialyl transferases within the context of the present invention include, but are not limited to, those sialyl transferases which catalyze the complex sialylation and branching of the N-and O-linked oligosaccharides.

The overall content of sialic acid in the glycoprotein may be affected by controlling cell culture parameters which affect cell specific productivity (see, for example, U.S. Pat. No. 5,705,364). Factors which affect cell specific productivity are well known in the art and include, but are not limited to, factors which affect DNA/RNA copy number, factors which affect RNA, such as factors which stabilize RNA, media nutrients and other supplements, the concentration of transcription enhancers, the osmolality of the culture environment, the temperature and pH of the cell culture, and the like.

For the culture of the mammalian cells expressing the desired protein and capable of adding the desired carbohydrates in specific position and linkage, numerous culture conditions can be used paying particular attention to the host cell being cultured. Suitable culture conditions for mammalian cells are well known in the art (J. Immunol. Methods (1983) 56:221-234) or can be easily determined by the skilled artisan (see, for example, Animal Cell Culture: A Practical Approach 2nd Ed., Rickwood, D. and Hames, B. D., eds. Oxford University Press, New York (1992)), and vary according to the particular host cell selected.

The mammalian cell culture is prepared in a medium suitable for the particular cell; being cultured. Commercially available media such as Ham's F10 (Sigma), Minimal Essential Medium (MEM, Sigma), RPMI-1640 (Sigma), and Dulbecco's Modified Eagle's Medium (DMEM, Sigma) are exemplary nutrient solutions. In addition, any of the media described in Ham and Wallace, (1979) Meth. Enz., 58:44; Bames and Sato, (1980) Anal. Biochem., 102:255; U.S. Pat. Nos. 4,767,704; 4,657,866; 4, 927,762; 5,122,469 or 4,560,655; International Publication Nos. WO 90/03430; and WO 87/00195; may be used as culture media. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin® drug), trace elements (defined as inorganic compounds usually present at final concentrations in the micromolar range) lipids (such as linoleic or other fatty acids) and their suitable carriers, and glucose or an equivalent energy source. Any other necessary supplements may also be included at appropriate concentrations that would be known to those skilled in the art.

The glycoproteins may be produced by growing cells which express the desired protein under a variety of cell culture conditions. For instance, cell culture procedures for the large or small scale production of proteins are potentially useful within the context of the present invention. Procedures including, but not limited to, a fluidized bed bioreactor, hollow fiber bioreactor, roller bottle culture, or stirred tank bioreactor system may be used, in the later two systems, with or without microcarriers, and operated alternatively in a batch, fed-batch, or continuous mode.

In some embodiments the cell culture of the present invention is performed in a stirred tank bioreactor system and a fed batch culture procedure is employed. In the preferred fed batch culture the mammalian host cells and culture medium are supplied to a culturing vessel initially and additional culture nutrients are fed, continuously or in discrete increments, to the culture during culturing, with or without periodic cell and/or product harvest before termination of culture. The fed batch culture can include, for example, a semi-continuous fed batch culture, wherein periodically whole culture (including cells and medium) is removed and replaced by fresh medium. Fed batch culture is distinguished from simple batch culture in which all components for cell culturing (including the cells and all culture nutrients) are supplied to the culturing vessel at the start of the culturing process. Fed batch culture can be further distinguished from perfusion culturing insofar as the supemate is not removed from the culturing vessel during the process (in perfusion culturing, the cells are restrained in the culture by, e.g., filtration, encapsulation, anchoring to microcarriers, sedimentation, etc. and the culture medium is continuously or intermittently introduced and removed from the culturing vessel).

Further, the cells of the culture may be propagated according to any scheme or routine that may be suitable for the particular host cell and the particular production plan contemplated. Therefore, the present invention contemplates a single step or multiple step culture procedure. In a single step culture the host cells are inoculated into a culture environment and the processes of the instant invention are employed during a single production phase of the cell culture. Alternatively, a multi-stage culture is envisioned. In the multi-stage culture cells may be cultivated in a number of steps or phases. For instance, cells may be grown in a first step or growth phase culture wherein cells, possibly removed from storage, are inoculated into a medium suitable for promoting growth and high viability. The cells may be maintained in the growth phase for a suitable period of time by the addition of fresh medium to the host cell culture.

According to some embodiments, fed batch or continuous cell culture conditions are devised to enhance growth of the mammalian cells in the growth phase of the cell culture. In the growth phase cells are grown under conditions and for a period of time that is maximized for growth. Culture conditions, such as temperature, pH, dissolved oxygen (dO₂) and the like, are those used with the particular host and will be apparent to the ordinarily skilled artisan. Generally, the pH is adjusted to a level between about 6.5 and 7.5 using either an acid (e.g., CO₂) or a base (e. g., Na₂CO₃ or NaOH). A suitable temperature range for culturing mammalian cells such as CHO cells is between about 30 to 38° C. and preferably about 37° C. and a suitable dO₂ is between 5-90% of air saturation.

At a particular stage the cells may be used to inoculate a production phase or step of the cell culture. Alternatively, as described above the production phase or step may be continuous with the inoculation or growth phase or step.

According to the present invention, the cell culture environment during the production phase of the cell culture is controlled. According to the process of the present invention, the zinc ion, cobalt ion or IGF-1concentration in the culture medium is manipulated such that the desired sialic acid content is achieved and maintained in the resulting glycoprotein. In some aspects, the production phase of the cell culture process is preceded by a transition phase of the cell culture in which parameters (such as addition of zinc ion, cobalt ion or IGF-1) for the production phase of the cell culture are engaged.

According to the present invention the concentration of zinc ion, cobalt ion, or IGF-1 is controlled to control desialylation resulting in increased sialic acid in the glycoprotein recovered from the process of the invention. Concentrations of zinc and cobalt ions, as well as IGF-1, are chosen keeping in mind other process parameters such as the osmolality of the production phase which can affect the cell specific productivity and sialic acid content of the glycoprotein produced.

The present invention provides, in a particular embodiment, for producing a glycoprotein in mammalian cell culture by adding an effective amount of Zn²⁺ or Co²⁺ to a culture medium in which cells producing the glycoprotein are maintained.

Thus, in some embodiments, provided are methods or processes for producing a glycoprotein in mammalian cell culture by adding an effective amount of Co²⁺ to a culture medium in which cells producing the glycoprotein are grown. In some embodiments, the effective amount is enough to inhibit sialidase expression and/or activity. Thus, in some embodiments, the concentration of Co²⁺ is between approximately 0.005 mM and 50 mM. The foregoing parameter is controlled to affect the mature glycoprotein sialic acid content. In some embodiments, the Co²⁺ is added in the form of a cobalt salt. In some embodiments, the Co²⁺ is derived from compounds selected from the group consisting of CoCl₂ and CoSO₄.

Similarly, in some embodiments, methods or processes for producing a glycoprotein in mammalian cell culture by adding an effective amount of Zn²⁺ to a culture medium in which cells producing the glycoprotein are grown to inhibit sialidase expression and/or activity. In some embodiments, the effective amount is enough to inhibit sialidase expression and/or activity. Thus, in some embodiments, the concentration of Zn²⁺ is between approximately 0.005 mM and 50 mM. The foregoing parameter is controlled to affect the mature glycoprotein sialic acid content. In some embodiments, the Zn²⁺ is added in the form of a cobalt salt. In some embodiments, the Zn²⁺ is derived from compounds selected from the group consisting of ZnCl₂ and ZnSO₄.

In some embodiments, the concentration of Zn²⁺ or Co²⁺ may be, for example, greter than one or more of the following values: 0.005 mM, 0.006 mM, 0.007 mM, 0.008 mM, 0.009 mM, 0.01 mM, 0.02 mM, 0.03 mM, 0.04 mM, 0.05 mM, 0.06 mM, 0.07 mM, 0.08 mM, 0.09 mM, 0.10 mM, 0.11 mM, 0.12 mM, 0.13 mM, 0.14 mM, 0.15 mM, 0.2 mM, 0.25 mM, 0.30 mM, 0.35 mM, 0.40 mM, 0.5 mM, 1.0 mM, 5.0 mM, 10.0 mM, 15.0 mM, 20.0 mM, 25.0 mM, 30.0 mM, 35.0 mM, 40.0 mM, 45.0 mM, 50.0 mM.

The zinc ion is added by any means known in the art, such as by the addition of ZnCl₂. In a preferred embodiment the zinc is added in batch to the fed batch culture system with or without other appropriate nutrients as described herein or known to those skilled in the art of mammalian cell culture.

The cobalt ion is added by any means known in the art, such as by the addition of CoCl₂. In a preferred embodiment the cobalt is added in batch to the fed batch culture system with or without other appropriate nutrients as described herein or known to those skilled in the art of mammalian cell culture.

Also provided in some embodiments are methods or processes for producing a glycoprotein in a mammalian cell culture, such as in a baby hamster kidney (BHK) cell culture, by adding an effective amount of insulin-like growth factor 1 (IGF-1) to a cell culture medium. In some embodiments, the levels of IGF-1 useful to reduce expression and/or activity of sialidase is selected from the following ranges: 1-10 ng/ml, 1-5 ng/ml, 5-10 ng/ml, 10-20 ng/ml, 10-15 ng/ml, 15-20 ng/ml, 20-30 ng/ml, 20-25 ng/ml, 25-30 ng/ml, 30-40 ng/ml, 30-35 ng/ml, 35-40 ng/ml, 40-50 ng/ml, 40-45 ng/ml, 45-50 ng/ml, 50-60 ng/ml, 60-70 ng/ml, 70-80 ng/ml, and 80-90 ng/ml.

Alternatively, for other mammalian host cells and other glycoproteins, small test cultures can be prepared and sialic acid content of the glycoprotein may be ascertained at various zinc or cobalt ion concentrations or IGF-1 concentrations appropriate for the particular host cell being cultured and the particular phase of the culture. In some embodiments, the zinc ion, cobalt ion, or IGF-1 is added to the host cell culture at or about the time the production phase of the cell culture is initiated. Conveniently, a transition phase is employed during the cell culture process preceding the production phase in which the cell culture conditions as discussed herein are engaged for the desired increase in sialic acid content and hence the desired glycoform profile. At this time the temperature of the culture may also be shifted, which in some embodiments is to between about 30° C and 36° C, or to about 33° C.

In some embodiments, the methods can be further optimized by a combination of techniques used above to increase sialic acid capping. In some embodiments, zinc ions and IGF-1 can be added to the cell culture medium. In some embodiments, cobalt ions and IGF- ions can be added to the cell culture medium. In other embodiments, zinc and cobalt ions can be added to the cell culture medium.

Additionally, the methods can be further optimized by altering the pH of the cell culture medium to reduce the sialidase activity. In some embodiments, particularly for BHK cells, the pH is 6.0 or below, 6.5 or below, 7.0 or below, 6.0 to 7.0, and or 6.5 to 7.0.

The methods and processes are believed to be useful with any recombinantly produced glycoprotein. Examples are hormones such as insulin, growth hormones (including human growth hormone and bovine growth hormone), tissue-type plasminogen activator (t-PA), rennin, clotting factors such as factor VII, factor VIII and factor IX, bombesin, thrombin, hemopoietic growth factor, serum albumin, receptors for hormones or growth factors, interleukins, colony stimulating factors, T-cell receptors, MHC polypeptides, viral antigens, glycosyltransferases, and the like. Other glycoproteins useful in the methods of the invention include α1-antitrypsin, erythropoietin, granulocyte-macrophage colony stimulating factor, anti-thrombin III, interleukin 6, interferon β (beta), protein C, fibrinogen, among many others. This list of glycoproteins is exemplary, not exclusive and also those include in the Definition section listed above.

It will be understood by the skilled artisan that the cell culture procedures of the present invention are selected to achieve the desired level of sialylation of the produced protein. Process parameters in addition to those described herein which influence the degree of sialylation include oxygen level, ammonium level, pH and hexose level. Culture density, time and storage conditions such as temperature also influence sialylation. The present invention is meant to include those process parameters which are additionally most suitable for enhanced sialylation. Additionally, for example, U.S. Patent No. 5,705,364 provides methods of altering the sialic acid content of a glycoprotein by controlling factors that affect productivity, such as the addition of an alkanoic acid to the culture medium, controlling osmolality of the culture medium, and controlling growth temperature.

### III. Recovery of the Glycoprotein

Following the polypeptide production phase, the polypeptide of interest is recovered from the culture medium using techniques which are well established in the art.

The polypeptide of interest preferably is recovered from the culture medium as a secreted polypeptide. For example, as a first step, the culture medium or lysate is centrifuged to remove particulate cell debris. The polypeptide thereafter is purified from contaminant soluble proteins and polypeptides, with the following procedures being exemplary of suitable purification procedures: by fractionation on immunoaffinity or ion-exchange columns; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and protein A Sepharose columns to remove contaminants such as IgG.

### IV. Analysis of the Glycoprotein

The complex carbohydrate portion of the glycoprotein produced by the processes described herein may be readily analyzed if desired, by conventional techniques of carbohydrate analysis. Thus, for example, techniques such as lectin blotting, well-known in the art, reveal proportions of terminal mannose or other sugars such as galactose. Termination of mono-, bi-, tri-, or tetra-antennary oligosaccharide by sialic acids can be confirmed by release of sugars from the protein using anhydrous hydrazine or enzymatic methods and fractionation of oligosaccharides by ion-exchange or size exclusion chromatography or other methods well-known in the art. The isoelectric point (pl) of the glycoprotein can also be measured, before and after treatment with neuraminidase to remove sialic acids. An increase in pl following neuraminidase treatment indicates the presence of sialic acids on the glycoprotein.

The resulting carbohydrates can be analyzed by any method known in the art including those methods described herein. Several methods are known in the art for glycosylation analysis and are useful in the context of the present invention. Such methods provide information regarding the identity and the composition of the oligosaccharide attached to the peptide. Methods for carbohydrate analysis useful in the present invention include but are not limited to lectin chromatography; HPAEC-PAD, which uses high pH anion exchange chromatography to separate oligosaccharides based on charge; NMR; Mass spectrometry; HPLC; GPC; monosaccharide compositional analysis; sequential enzymatic digestion.

Sialic acid can be determined separately by the direct calorimetric method of Yao et al. (Anal Biochem. 179:332-335 (1989)) in triplicate samples. In a preferred embodiment the thiobarbaturic acid (TBA) of Warren, L. J. Biol Chem 238:(8) (1959) or the method of Anumula, K. R., (1995) Anal. Biochem. 230:24-30 is used.

Having now generally described the invention, the same will be more readily understood through reference to the following examples which are provided by way of illustration, and are not intended to be limiting of the present invention, unless specified.

### EXAMPLES

It will be apparent to those skilled in the art that the examples and embodiments described herein are by way of illustration and not of limitation, and that other examples may be used without departing from the spirit and scope of the present invention, as set forth in the claims.

### Example 1. Materials and Methods

The experiments were performed in 15L fermenters with a 12L working volume using a proprietary medium ("X medium"), however other media are contemplated for use including those which are commercially available. Samples were taken from the fermenters at various time points and the fermenter samples were spun at 1000x gravity for 10 minutes. Clear supernatant was transferred to new tubes and cell pellets were lysed in M-PER (Pierce, Rockford, IL) or Phosphosafe lysis buffer (Novagen, Madison, WI) per instructions provided by vendor. Sialidase activity was quantitated using a modified NA-Star kit (Applied Biosystems, Foster City, CA) and Modulus microplate reader (Turner Biosystems, Sunnyvale, CA). LY294002, PD98059, Rapamycin and insulin-like growth factor 1 (IGF-1) were purchased from SAFC (Saint Louis, MO). For the detection of sialidase in immunoblots, a polyclonal antibody was used (Abnova Inc., Taiwan). A mouse monoclonal antibody (Santa Cruz Biotechnology, Santa Cruz, CA) and a polyclonal antibody (Cell Signaling Technology, Danvers, MA) were used to detect phosphoylated ERK and phosphorylated Akt, respectively.

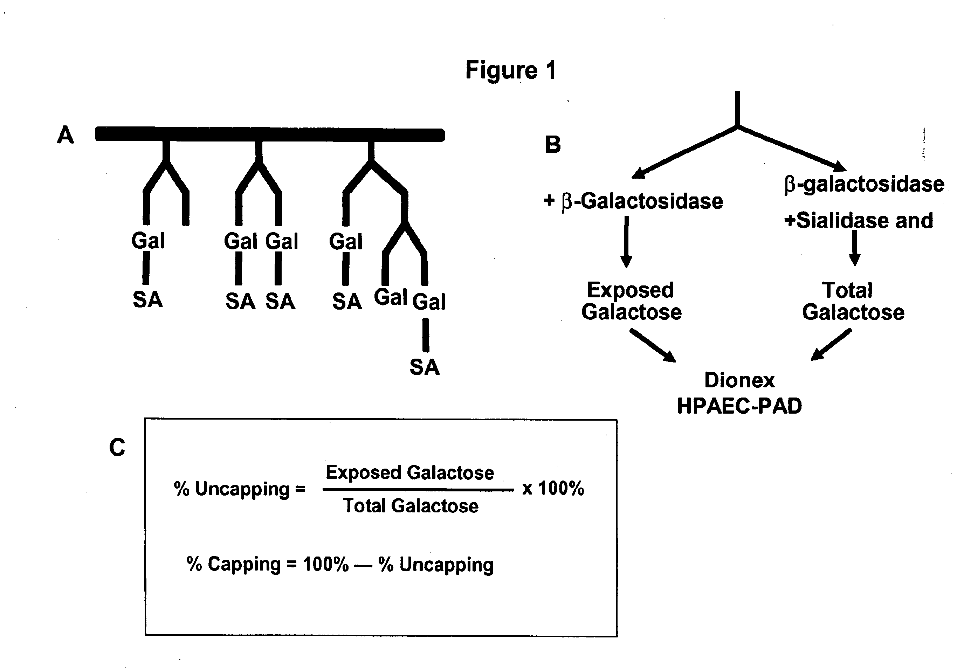

The purified glycoprotein K was subjected to sialidase and/or beta-galactosidase digestion and total and exposed galactose were measured using Dionex HPAEC-PAID. The formula shown in Figure 1 is used to calculation of capping percentage.

### Example 2. Low Capping vs. High Capping Cell Sources

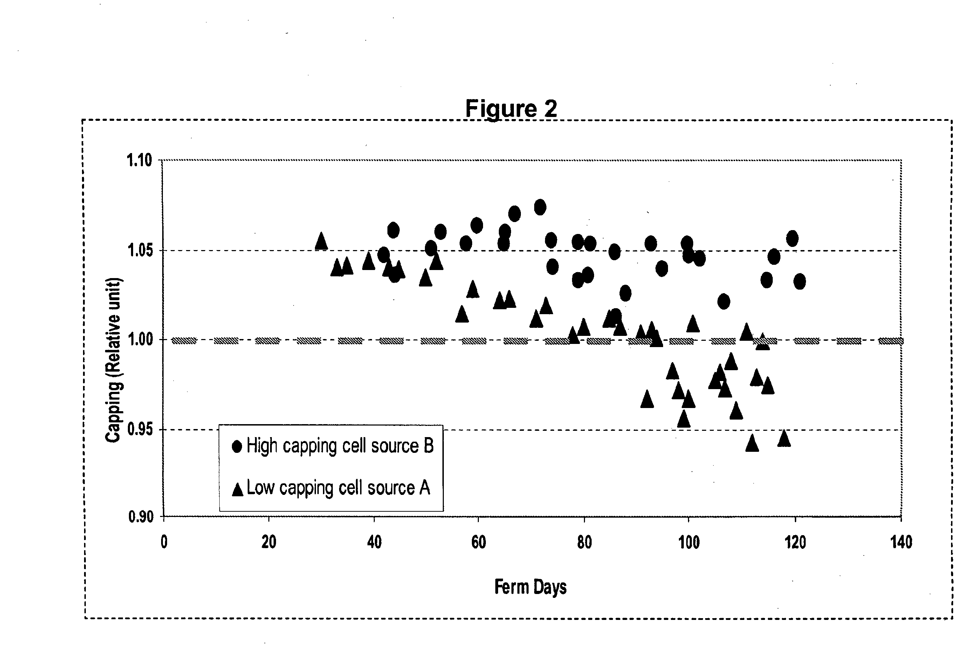

Different cell sources, even if from the same parent colony, may differ in sialic acid capping trend of the oligosaccharides. The disparities in such cell sources can greatly affect the production of a glycoprotein. Referring now to Figure 2, the cell source with low capping values resulted in downward trending of capping toward the middle of the campaign, whereas the one rated as a high-capping cell source maintained the capping values above the limit during the full campaign.

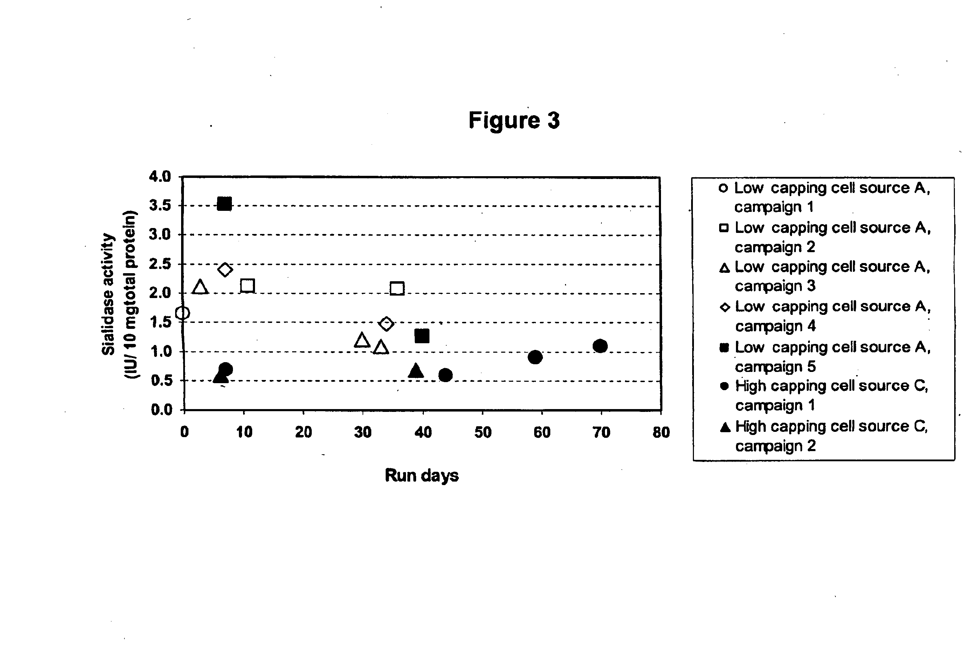

Thus, cell sources play an important part in sialic acid capping of oligosaccharides which may be attributed to sialidase activity. Shown in Figure 3 is a chart depicting that the cell source with low capping values consistently showed elevated sialidase activity in cell lysate in different commercial-scale manufacturing campaigns.

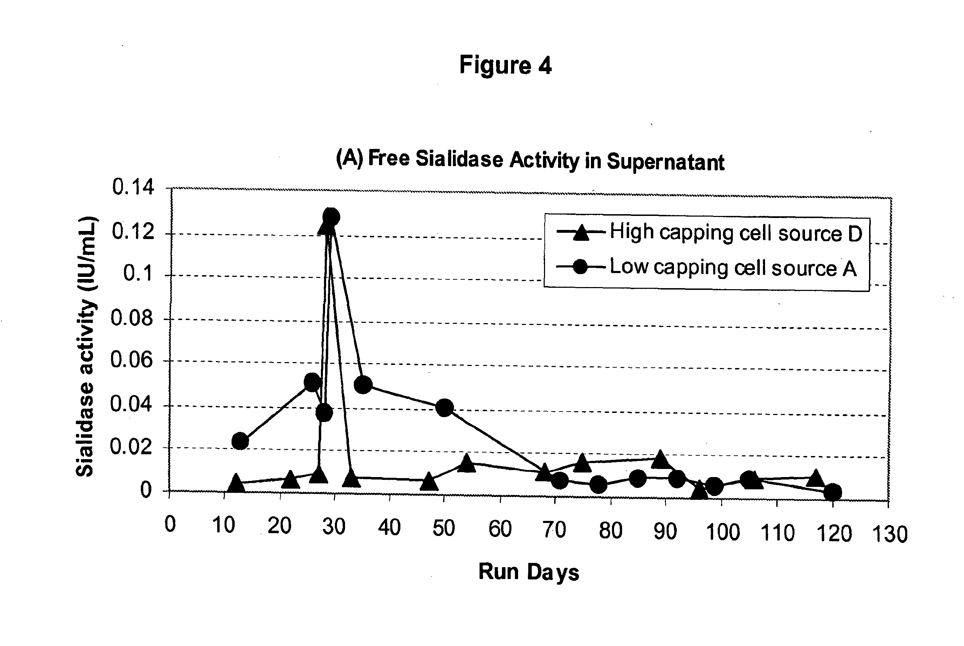

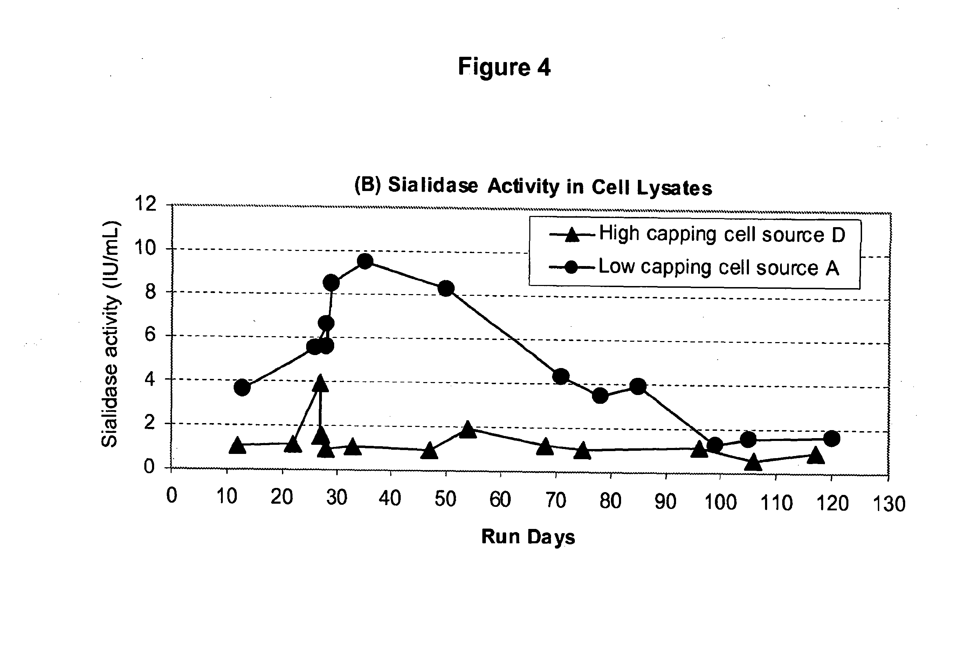

High sialidase activity was observed in harvested cell culture fluid as well as cell lysate in 15L bioreactor fermentation using the cell source with historically low capping values, as shown in Figures 4(A) and 4(B). It is interesting to note the detection of acute response of sialidase activity in cell lysate and cell culture fluids to galactose supplementation (3 g/L) in the medium at day 29 in both cell sources.

### Example 3. Insulin-like Growth Factor 1 (IGF-1) Inhibits Sialidase Activity

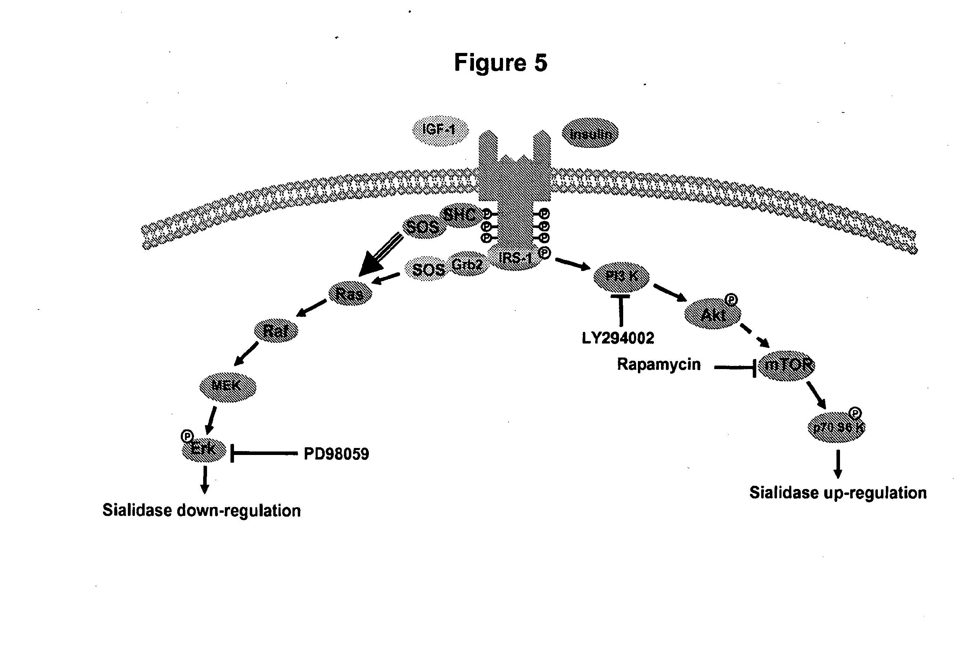

Insulin-like growth factor 1 (IGF-1) and insulin receptors auto-phosphorylation activates, through insulin receptor substrate 1 (IRS-1) recruitment, different downstream signals and trigger both cell proliferation and differentiation. Shown in Figure 5 is the IGF-1/insulin receptor pathway. Activation of the Ras-Raf-Ker-Mek pathway stimulates proliferation, which contributes to sialidase down-regulation. On the contrary, activation of PI3K-Akt-mTOR-P70S6K pathway leads to sialidase expression. It is important to note that only the IGF-1 receptor is capable of mediating IRS-1 independent activation of the Erk pathway.

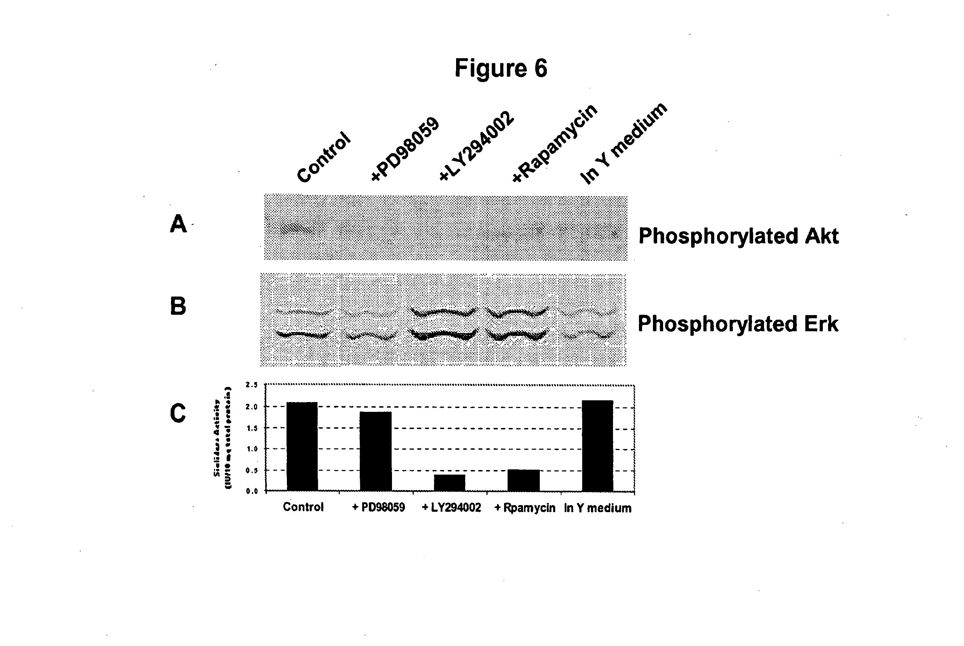

BHK cells were cultured in medium supplemented with different inhibitors in roller bottles for 6 days. The cell lysate was prepared using Phosphosafe lysis buffer (Novagen, Madison, WI) and was subjected to immunoblots using appropriate antibodies. The results, shown in Figure 6, indicated that the Erk phosphorylation was prevented in the presence of 15 µM PD98059, and Akt phosphorylation in the presence of 20 µM LY294002 or 5 ng/mL rapamycin. Suppression of Akt activation by LY294002 or rapamycin further resulted in Erk phosphorylation and down-regulation of sialidase expression in BHK cells. It was also observed that BHK cells cultured in another proprietary medium ("Y medium") was shown to in-activate the Erk phosphorylation. However no difference on sialidase activity was found compared to control. This is consistent with the observation of that the inhibition of Erk activation by PD98059 didn't result in change of sialidase expression.

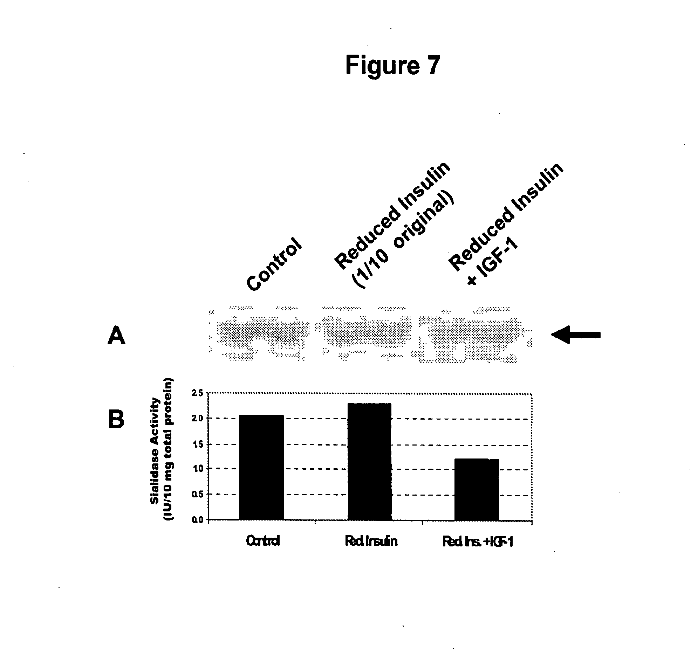

Reduction of insulin (10 fold) in the medium was shown to have no effect on the sialidase activity in BHK cells cultured in roller bottles, shown in Figure 7. The addition of IGF-1 (20 ng/mL) in the medium with reduced insulin down-regulated sialidase expression compared to control.

### Example 4. Sialidase Activity is pH Dependent

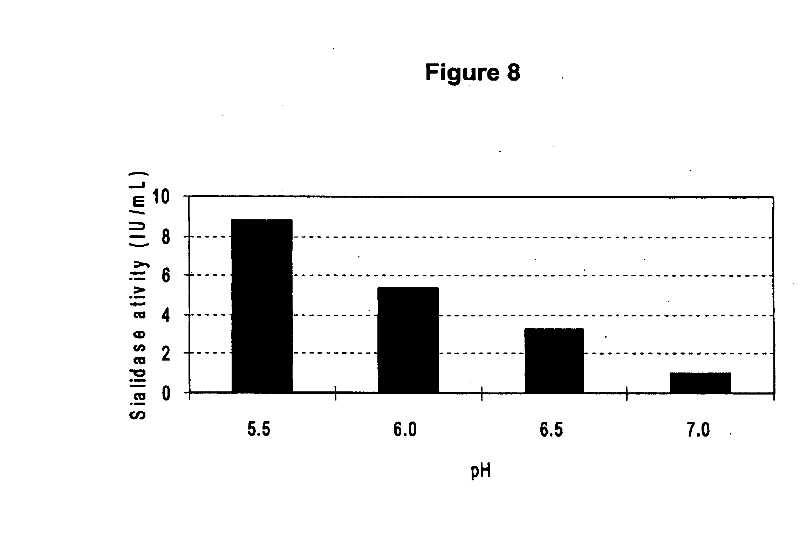

It has also been shown that sialidase activity in BHK cells is pH dependent, shown in Figure 8. Cell lysate was prepared using M-PER lysis buffer (Pierce) and sialidase activity was measured using modified NA-Star kit (Applied Biosystems) under different pH conditions. It was found that the sialidase activity in BHK cells was shown to have a higher activity at acidic pH.

### Example 5. Inhibition of Sialidase Activity using Trace Elements

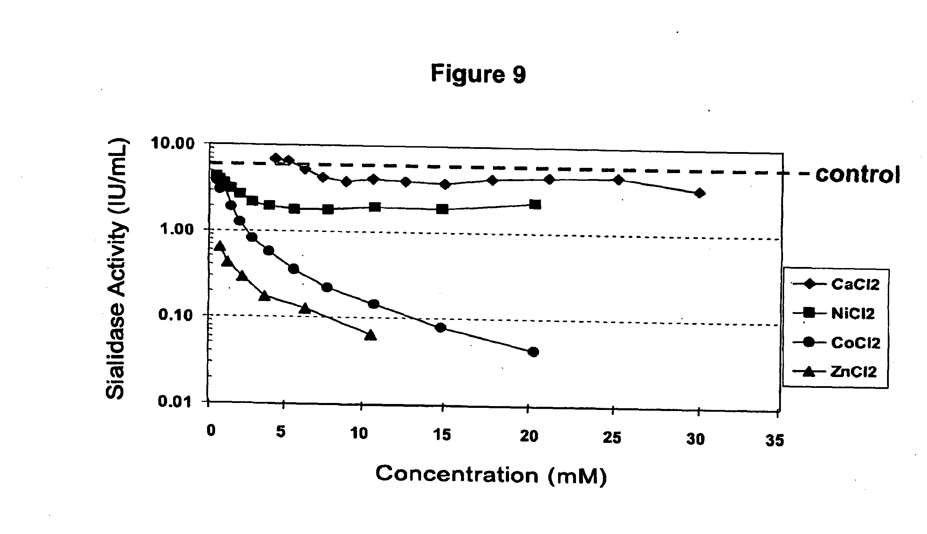

Referring now to Figure 9, shown is the inhibition of sialidase activity using trace elements, including CaCl₂, NiCl₂, CoCl₂, and ZnCl₂. Cell lysate of BHK cells prepared using M-PER lysis buffer (Pierce) was treated with different trace elements at various concentrations. The data indicated that ZnCl₂ and CoCl₂ inhibit the sialidase activity effectively *in vitro.*

## Claims

1. A method of increasing the percentage of oligosccharides terminating in sialic acid in recombinantly produced glycoproteins comprising inhibiting expression and/or activity of sialidase in a recombinant cell culture, wherein inhibition of the expression and/or activity of sialidase comprises addition of an effective amount of insulin-like growth factor 1 (IGF-1) to the recombinant cell culture.

2. The method of claim 1, wherein inhibition of the expression and/or activity of sialidase further comprises addition of an effective amount of zinc ion, cobalt ion, or both to the recombinant cell culture..

3. The method of claim 2, wherein the zinc ion or cobalt ion has a concentration between 0.005 mM and 50 mM.

4. The method of claim 2, wherein the zinc ion is added in a form of a zinc salt.

5. The method of claim 4, wherein the zinc salt is selected from the group consisting of ZnCl₂ and ZnSO₄.

6. The method of claim 2, wherein the cobalt ion is added in a form of a cobalt salt.

7. The method of claim 6, wherein the cobalt salt is selected from the group consisting of CoCl₂ and CoSO₄.

8. The method according to any of claims 2-7, further comprising altering pH of the cell culture.

9. The method of claim 8, wherein the pH 7.0 or below.

10. The method of claim 9, wherein the pH is 6.5-7.0.

11. The method of claim 11, wherein the IGF-1 has a concentration between 1-90 ng/ml.

## Patentansprüche

1. Verfahren zur Steigerung des Prozentanteils von in Sialinsäure endenden Oligosacchariden in rekombinant hergestellten Glycoproteinen, bei dem man die Expression und/oder Aktivität von Sialidase in einer rekombinanten Zellkultur hemmt, wobei die Hemmung der Expression und/oder Aktivität von Sialidase die Zugabe einer wirksamen Menge von IGF-1 (Insulin-like Growth Factor 1) zu der rekombinanten Zellkultur umfasst.

2. Verfahren nach Anspruch 1 , wobei die Hemmung der Expression und/oder Aktivität von Sialidase ferner die Zugabe einer wirksamen Menge an Zinkion, Cobaltion oder beiden zu der rekombinanten Zellkultur umfasst.

3. Verfahren nach Anspruch 2, wobei das Zinkion oder Cobaltion eine Konzentration zwischen 0,005 mM und 50 mM aufweist.

4. Verfahren nach Anspruch 2, wobei das Zinkion in einer Form eines Zinksalzes zugegeben wird.

5. Verfahren nach Anspruch 4, wobei das Zinksalz aus der aus ZnCl₂ und ZnSO₄ bestehenden Gruppe ausgewählt ist.

6. Verfahren nach Anspruch 2, wobei das Cobaltion in einer Form eines Cobaltsalzes zugegeben wird.

7. Verfahren nach Anspruch 6, wobei das Cobaltsalz aus der aus CoCl₂ und CoSO₄ bestehenden Gruppe aus-gewählt ist.

8. Verfahren nach einem der Ansprüche 2-7, bei dem man ferner den pH-Wert der Zellkultur verändert.

9. Verfahren nach Anspruch 8, wobei der pH-Wert bei 7,0 oder darunter liegt.

10. Verfahren nach Anspruch 9, wobei der pH-Wert bei 6,5-7,0 liegt.

11. Verfahren nach Anspruch 11, wobei der IGF-1 eine Konzentration zwischen 1-90 ng/ml aufweist.

## Revendications

1. Procédé pour augmenter le pourcentage d'oligosaccharides se terminant par de l'acide sialique dans des glycoprotéines produites de manière recombinante, comprenant l'inhibition de l'expression et/ou de l'activité de la sialidase dans une culture cellulaire recombinante, l'inhibition de l'expression et/ou de l'activité de la sialidase comprenant l'addition d'une quantité efficace de facteur de croissance 1 analogue à l'insuline (IGF-1) à la culture cellulaire recombinante.

2. Procédé selon la revendication 1, l'inhibition de l'expression et/ou de l'activité de la sialidase comprenant en outre l'addition d'une quantité efficace d'ions de zinc, d'ions de cobalt ou des deux à la culture cellulaire recombinante.

3. Procédé selon la revendication 2, la concentration en ions de zinc ou en ions de cobalt étant située entre 0,005 mM et 50 mM.

4. Procédé selon la revendication 2, les ions de zinc étant ajoutés sous forme d'un sel de zinc.

5. Procédé selon la revendication 4, le sel de zinc étant choisi dans le groupe constitué par ZnCl₂ et ZnSO₄.

6. Procédé selon la revendication 2, les ions de cobalt étant ajoutés sous forme d'un sel de cobalt.

7. Procédé selon la revendication 6, le sel de cobalt étant choisi dans le groupe constitué par CoCl₂ et CoSO₄.

8. Procédé selon l'une quelconque des revendications 2-7, comprenant en outre la modification du pH de la culture cellulaire.

9. Procédé selon la revendication 8, le pH étant de 7,0 ou moins.

10. Procédé selon la revendication 9, le pH étant de 6,5-7,0.

11. Procédé selon la revendication 11, la concentration en IGF-1 étant située entre 1-90 ng/ml.
